# EUROPEAN PATENT APPLICATION

(11) **EP 0 908 183 A1**
(43) Date of publication of application: **14.04.1999**
(21) Application number: 98118921.0
(22) Date of filing: 07.10.1998
(51) Int. Cl.: A61K 31/565

(54) **Dehydroepiandrosterone or derivatives thereof for increasing the content of hyaluronic acid in skin**

(30) Priority: 08.10.1997 JP 275870/97
(71) Applicant: INSTITUTE FOR ADVANCED SKIN RESEARCH INC., Yokohama-shi, Kanagawa-ken (JP)
(72) Inventor: Nishina, Hiromichi, Gotenba-shi, Shizuoka-ken (JP); Ishikawa, Yasuko, Hino-shi, Tokyo (JP); Matsushita, Hiroshi, Tokyo (JP)
(74) Representative: VOSSIUS & PARTNER

(57) **Abstract**

The skin that has developed symptoms of aging or on which a scar has formed in the process of injury healing is characterized by a lower content of hyaluronic acid. To ameliorate these symptoms, a drug is offered that is effective, with high safety, in retarding a decrease or promoting an increase in the content of hyaluronic acid in skin. The drug is used as an active ingredient at least one substance selected from the group consisting of dehydroepiandrosterone, dehydroepiandrosterone derivatives such as dehydroepiandrosterone sulfate and pharmaceutically acceptable salts of said derivatives.

## Description

This invention relates to a drug effective to increase the content of hyaluronic acid in skin. More particularly, the invention relates to a drug that either retards a decrease or promotes an increase in the content of hyaluronic acid in skin with limited side-effects. Hyaluronic acid is a causative factor in manifesting symptoms of aging of skin, and a contributory factor in scar formation in the treatment of skin injuries.

Symptoms of skin aging develop with the progress of age. Aging skin is characterized by a dry, firmless and wrinkled appearance. All efforts have long been made in vain to find a substance in skin that might be an etiological factor for skin to manifest those changes in its appearance.

Retinoic acid and estrogens have been known to be effective nosotropic agents that can treat and ameliorate the above-listed symptoms of aging skin. However, both therapeutics have serious side-effects, which are a major obstacle to their clinical application. Briefly, retinoic acid is teratogenic and estrogens act like female hormones. In addition, the administration of estrogens may exacerbate cardiovascular disorders, and is very risky to elderly persons. Under the circumstances, there exists a strong need for a therapeutic drug that can be safely used as an etiotropic drug.

Estrogens are known to be capable of increasing the content of hyaluronic acid in skin of young mice when injected subcutaneously or applied to skin. They are also known to be effective in preventing the aging of skin of human females after menopause. A major problem with estrogens is the side-effects caused by their inherent nature as female hormones. Therefore, it has been desired to develop a drug that has the same effect as estrogens without acting like female hormones and which is administrable for external application.

When an injury to skin is in the process of healing, a scar tends to form in adult but this is not the case with a fetus. The degree of scar formation is also very small in babies and infants. Therefore, an injury to adult's skin should be treated in such a way as to minimize scar formation in the process of healing and this is particularly desired from an aesthetic viewpoint.

Under these circumstances, it has been desired to develop a drug which, by preventing a decrease in the content of hyaluronic acid in skin or increasing its content, can prevent the aging of skin and minimize the scar formation during healing of an injury to skin to such a degree as to maintain it in a beautiful condition.

An object, therefore, of the invention is to provide a pharmaceutical drug that is effective in treating or preventing the symptoms of aging of skin. A need exists to develop a drug that minimizes the scar formation on skin of an adult when an injury to his or her skin is in the process of healing.

Another object of the invention is to provide a drug that can treat or prevent aging of skin with minimum side-effects.

A further object of the invention is to provide a therapeutic or preventive drug that can ameliorate the symptoms of aging of skin by an etiotropic approach based on the etiology and pathology of the symptoms and which is also capable of preventing scar formation.

With a view to solving the aforementioned problems of the prior art, the present inventors conducted intensive studies on an extracellular substrate of aging skin, particularly with respect to its pathophysiology. As a result, we found that dehydroepiandrosterone (DHEA), dehydroepiandrosterone derivatives such as dehydroepiandrosterone sulfate and pharmaceutically acceptable salts of such derivatives are effective for the stated purpose and the present invention has been accomplished on the basis of this finding.

Dehydroepiandrosterone is a steroid hormone secreted from the adrenal cortex and its blood level is known to decrease with aging. The present inventors found that topical application of dehydroepiandrosterone to skin increased the content of hyaluronic acid which was an extracellular substrate decreasing with aging.

Dehydroepiandrosterone is eventually converted to estrogen under the action of aromatase. On the other hand, the ability of dehydroepiandrosterone to increase the content of hyaluronic acid was not lowered even when it was used in combination with alimidex which inhibits an aromatase. It was therefore confirmed that dehydroepiandrosterone itself was a hyaluronic acid enhancer.

As a matter of fact, hyaluronic acid decreased in aging skin or at wrinkled sites. If wrinkles are remedied by an electromagnetic method, hyaluronic acid increases at the treated site. It is also known that skin becomes less firm as it ages and hyaluronic acid is substantially lost before the age of 60.

Topical application of retinoic acid is effective in eliminating wrinkles and this effect is known to result from an increase in skin content of hyaluronic acid. Using estrogens to restore firmness and youth in aging skin is already known and it is also known that upon application of estrogens, the hyaluronic acid in skin of a mouse can be increased to restore its youth.

These findings have led to the understanding that increasing the content of hyaluronic acid in skin is important for the purpose of preventing its aging or restoring its youth.

As already mentioned, there is little or no scar formation on skin of a fetus or baby in the process of the healing of an injury to their skin. The present inventors speculated that the high skin content of hyaluronic acid may be one of the reasons for this phenomenon. Continuing their studies based on this assumption, the inventors found that the scar formation during the healing of a wound could be sufficiently prevented to maintain skin in a beautiful condition by preventing a decrease in skin content of hyaluronic acid or by positively increasing its content.
Fig. 1 is a graph showing how the content of hyaluronic acid in skin increased when the DHEA of the invention and comparative drugs were applied to skin of aging mice; and
Fig. 2 is a graph showing how the content of hyaluronic acid in skin varied when the DHEA of the invention and an aromatase inhibitor were applied to skin of aging mice, the aromatase inhibitor being applied either individually or in combination with DHEA.

It was confirmed that dehydroepiandrosterone (DHEA), which is used as an active ingredient in the present invention, was effective in increasing the hyaluronic acid content of skin to prevent and treat the symptoms of aging of skin. It was also confirmed that dehydroepiandrosterone was effective in inhibiting scar formation during the healing of an injury to skin. In addition, unlike retinoic acid which is teratogenic and estrogens that induce cardiovascular disorders, DHEA in its stable sulfate form has been commercialized as a drug that aids the process of childbirth and is used in clinical applications. In acute toxicity tests with mice, the LD₅₀ of DHEA was at least 10 g/kg (peroral), 899 mg/kg (injected subcutaneously) and 274 mg/kg (injected intravenously); thus, DHEA has been found to be a very safe drug.

The DHEA which is used as an active ingredient in the invention can be formulated and processed into pharmaceutical preparations as drugs for external application or peroral drugs by any known methods. Preferred dosage forms are petrolatum-base ointments, emulsions, creams, lotions cataplasms and bath powders or cubes if DHEA is formulated as a drug for external application, as well as powders, granules, subtilized granules, tablets, capsules, suspensions and emulsions if DHEA is formulated as a peroral drug. If DHEA is to be used for preventing scar formation in the process of healing of an injury to skin, it may be incorporated in common drugs for treating scars by external application and thereafter applied topically.

The dose of administration of the DHEA of the invention varies with the site of administration, the degree of aging, and with the severity of an injury to be healed. Because of the extremely low level of side-effects it will cause, DHEA can be administered in comparatively high doses. A preferred daily dose in a human adult is from 18 to 0.01 g, with the range of 0.5 - 0.01 g being particularly preferred.

The following examples are provided for the purpose of further illustrating the present invention but they are in no way to be taken as limiting.

### Example 1: Increasing the content of hyaluronic acid in skin of aging mice by applying DHEA to skin

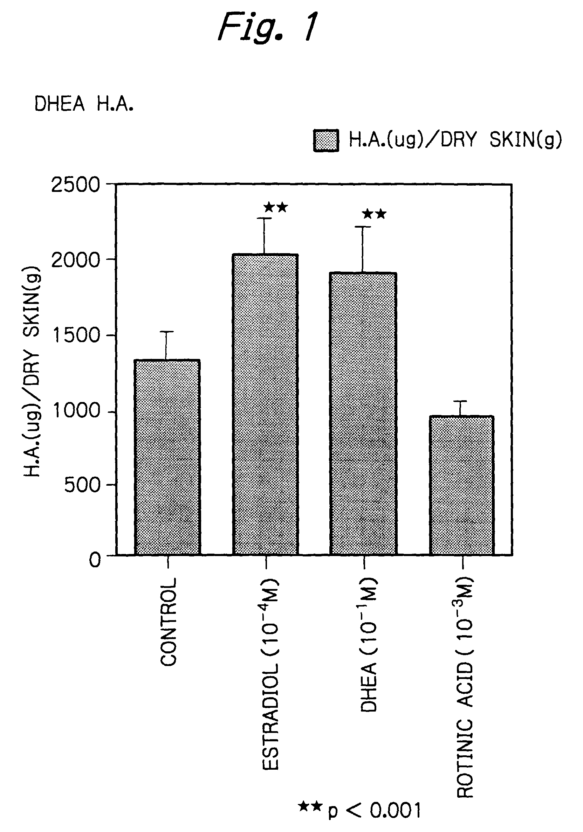

Dehydroepiandrosterone (DHEA) as an ethanol solution at a concentration of 10⁻¹M was applied to skin of the dorsal part of aging mice (Balb/c, 48 weeks old, in each group consisting of 6 animals) for 5 weeks. As well as estradiol (10⁻⁴M), DHEA increased the content of hyaluronic acid in skin with a statistically significant difference from the control group. On the other hand, retinoic acid that was tested simulaneously was not effective at all (see Fig. 1).

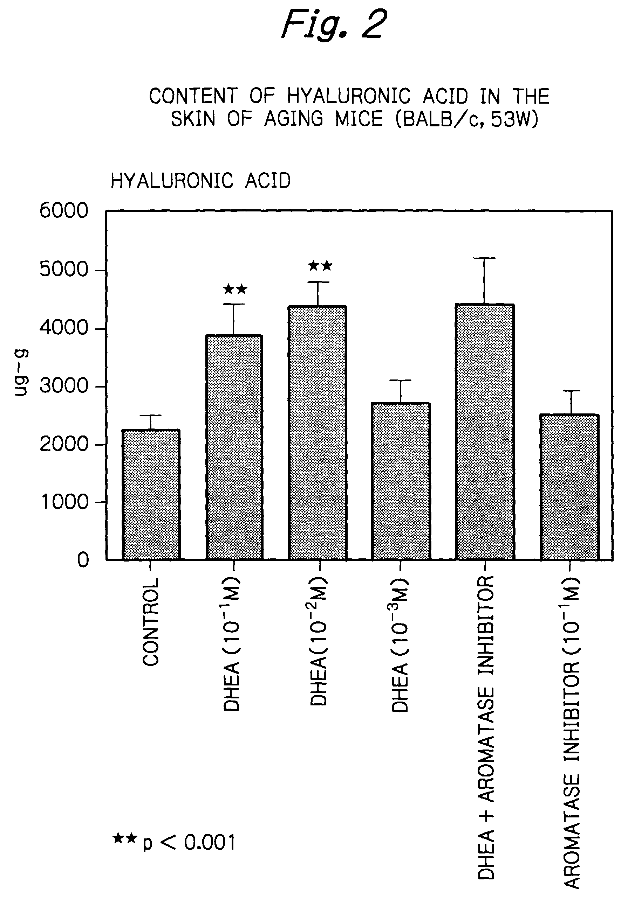

To evaluate the reproducibility of its efficacy, DHEA was applied in various concentrations of 10⁻¹, 10⁻² and 10⁻³M to aging mice (Balb/c, 53 weeks, in each group consisting of 8 animals). Further, in order to see whether DHEA became effective only after it was metabolized to estrodiol, alimidex that inhibited aromatase, which is an enzyme for causing a conversion to estrogens, was administered simultaneously with DHEA. The results were as follows: DHEA caused a statistically significant increase in the content of hyaluronic acid at concentrations of 10⁻¹M and 10⁻²M and it was found to have reproducible efficacy. This means the concentration of DHEA can be lowered to a tenth of the initial value. The aromatase inhibitor alimidex itself had no ability to increase the content of hyaluronic acid and the efficacy of DHEA was not suppressed even when it was combined with alimidex. Therefore, it was suggested that the hyaluronic acid increasing action of DHEA is not realized by its conversion to estrogens (see Fig. 2).

DHEA has already been known to have very low side-effects when administered to humans perorally or as a drug for external application. Hence, the above experimental results confirmed that DHEA is useful as a drug that shows an ability to increase the content of hyaluronic acid in skin.

### Example 2: Formulation (as ointment)

A hydrophilic base for ointment having the formulation indicated below was mixed with DHEA (6.2 g) of the invention and the mixture was processed to prepare an ointment.

| | |
|---|---|
| White petrolatum | 250 g |
| Stearyl alcohol | 220 g |
| Propylene glycol | 120 g |
| Sodium laurate | 15 g |
| Ethyl paraoxybenzoate | 0.25 g |
| Propyl paraoxybenzoate | 0.15 g |

## Claims

1. An agent for promoting an increase in the content of hyaluronic acid in skin which contains as an active ingredient at least one substance selected from the group consisting of dehydroepiandrosterone, dehyroepiandrosterone derivatives and pharmaceutically acceptable salts of said derivatives.

2. The agent according to claim 1, which is in a dosage form suitable for external application.

3. The agent according to claim 1, which is in a dosage form suitable for peroral administration.

4. The agent according to claim 1, 2 or 3, which is effective in preventing aging of skin or scar formation on the skin by promoting an increase in the content of hyaluronic acid in skin.

5. Use of at least one substance selected from the group consisting of dehydroepiandrosterone, dehydroepiandrosterone derivatives and pharmaceutically acceptable salts of said derivatives for the purpose of promoting an increase in the content of hyaluronic acid in skin.

6. Use of at least one substance selected from the group consisting of dehydroepiandrosterone, dehydroepiandrosterone derivatives and pharmaceutically acceptable salts of said derivatives for the preparation of a pharmaceutical composition for preventing the aging of skin or minimizing the scar formation during healing of an injury to skin.
